# EUROPEAN PATENT SPECIFICATION

(11) **EP 3 764 896 B1**
(45) Date of publication and mention of the grant of the patent: **16.03.2022**
(21) Application number: 19701735.3
(22) Date of filing: 22.01.2019
(51) Int. Cl.: A61B 5/08, A61B 5/00, G06K 9/00, A61B 5/113

(54) **METHOD AND APPARATUS FOR MONITORING A HUMAN OR ANIMAL SUBJECT**
VERFAHREN UND VORRICHTUNG ZUR ÜBERWACHUNG EINES MENSCHEN ODER TIERES
PROCÉDÉ ET APPAREIL POUR SURVEILLER UN SUJET HUMAIN OU ANIMAL

(30) Priority: 13.03.2018 GB 201803994
(43) Date of publication of application: 20.01.2021
(73) Proprietor: Oxford University Innovation Limited, Oxford, Oxfordshire OX2 0JB (GB)
(72) Inventor: JARCHI, Delaram, Oxford, Oxfordshire OX3 7EJ (GB); CLIFTON, David, Oxford, Oxfordshire OX3 0JS (GB); TARASSENKO, Lionel, Horton cum Studley, Oxfordshire OX33 1BN (GB)
(74) Representative: J A Kemp LLP
(86) International application number: PCT/GB2019/050164
(87) International publication number: WO 2019/175529

(56) References cited:
- WO-A1-2017/140663
- WO-A1-2017/140696
- WO-A1-2017/194915
- US-A1- 2017 071 547
- US-A1- 2017 325 779
- FOAD GHADERI ET AL: "Localizing Heart Sounds in Respiratory Signals Using Singular Spectrum Analysis", IEEE TRANSACTIONS ON BIOMEDICAL ENGINEERING, IEEE SERVICE CENTER, PISCATAWAY, NJ, USA, vol. 58, no. 12, 1 December 2011 (2011-12-01), pages 3360-3367, XP011371351, ISSN: 0018-9294, DOI: 10.1109/TBME.2011.2162728

## Description

The invention relates to monitoring a subject, particularly for extracting a frequency of a periodic physiological process such as a respiration rate.

Intelligent monitoring of vital signs (such as heart rate, respiratory rate (RR), blood oxygen saturation level (SpO2) and blood pressure) in unobtrusive ways is desirable to recognize patients at risk of deterioration. Replacement of manual recordings of physiological parameters (which are not always accurate or repeatable) by automated systems is desirable.

Automation of RR measurements while achieving high patient compliance and accuracy has proved challenging. RR is currently measured in hospitals by manual counting of breaths, conventional impedance pneumography (IP) or via an optical technique called end-tidal carbon dioxide (et-CO2). For continuous monitoring of RR, both IP and et-CO2 are subject to limitations in terms of patient compliance and accuracy.

Acceleration signals have been used for many motion analysis applications such as gait, posture, and balance analysis. The use of accelerometers for motion analysis has significantly increased due to their ease of use, portability, and ability to be integrated in low-power wireless embedded platforms. It has been proposed to using acceleration signals to monitor RR.

To estimate respiratory rate based on acceleration signals, a low-pass or band-pass filtering is typically applied to each axis separately. A frequency in the range of 0.05 Hz to 1 Hz with maximum peak amplitude in the spectral domain can then be selected to calculate the breaths per minute (bpm). A number of studies [1], [2], [3], [4] have investigated the estimation of respiratory rate from acceleration signals. A gas pressure system has been used to validate estimation of respiratory rate from accelerometers placed on the subject's sternum [5]. A relative mean error of -0.15 bpm has been reported. In [6], chest worn accelerometers were used to estimate respiratory rate and showed high correlation to flow rate measurements from a nasal cannula. A single chest patch sensor containing ECG and accelerometers has been used in [7] to compare fusion of three respiratory rate estimates, one estimate from accelerometer and two estimates using ECG from respiratory sinus arrhythmia and QRS modulation, to a capnography derived reference [8].

US 2017/325779A1 discloses a system and method capable of indirectly monitoring respiratory and cardiac variables. A decomposition technique on time-series of features extracted from the chest audio signal α(t) is proposed. The proposed monitoring system may acquire the acoustic signal on the chest of a subject by means of a wearable transducer.

WO 2017/194915 A1 discloses systems for determining the frequency of a periodic physiological process of a subject, particularly respiration rate or heart rate. In one arrangement plural time windows of physiological data are obtained. Reference features corresponding to modulation modes are identified. Modulations of the reference features are extracted. Quality parameters are obtained by processing the extracted modulations. The quality parameters represent how strongly the extracted modulation exhibits a waveform of the periodic physiological process. The extracted modulations are processed to calculate the frequency of the periodic physiological process of the subject.

FOAD GHADERI ET AL, "Localizing Heart Sounds in Respiratory Signals Using Singular Spectrum Analysis", IEEE TRANSACTIONS ON BIOMEDICAL ENGINEERING, IEEE SERVICE CENTER, PISCATAWAY, NJ, USA, (20111201), vol. 58, no. 12, doi:10.1109/TBME.2011.2162728, ISSN 0018-9294, pages 3360 - 3367 discloses use of singular spectrum analysis (SSA). Despite frequency overlap of heart and lung sound components, two different trends in the eigenvalue spectra are recognizable, which leads to find a subspace that contains more information about the underlying heart sound. Artificially mixed and real respiratory signals are used for evaluating the performance of the method.

WO 2017/140696 A1 discloses a device having a signal input for obtaining one or more sensor signals representing orientation changes and/or motion of a sensor. The device comprises a selector for determining combination values of said one or more sensor signals and for selecting one or more of said sensor signals either having the most extreme combination values or that most likely coincide with the direction of a breathing motion, a converter for converting the selected one or more sensor signals into the frequency domain to obtain one or more frequency-domain sensor signals, and a peak detector for performing maximum peak detection in said one or more frequency-domain sensor signals within a predetermined frequency range and selecting the frequency corresponding to the detected maximum peak as breathing rate.

WO 2017/140663 A1 discloses a data processing device that determines heart rate information regarding a subject of interest from time-dependent sensor data that includes physiological sensor data comprising a heartbeat component and at least one motion artifact component, and that further includes motion sensor data. A reconstruction unit receives artifact-removed physiological sensor data and decomposes it into a plurality of artifact- removed physiological sensor data components with associated component amplitudes, and provides reconstructed heartbeat component data as a combination of a component subset of at least two of the artifact-removed physiological sensor data components of highest component amplitudes. A beat analysis unit receives the reconstructed heartbeat component data and determines an interbeat interval from the heartbeat component data as the heart rate information, and provides a heart rate information signal based on the determined heart rate information.

US 2017/071547 A1 discloses a data processing device is disclosed for extracting a desired vital signal containing a physiological information component from sensor data that includes time-dependent first sensor data comprising the physiological information component and at least one motion artifact component, and that includes time-dependent second sensor data that is indicative of a position, a velocity or an acceleration of the sensed region as a function of time. A decomposition unit decomposes the second sensor data into at least two components of decomposed sensor data and, based on the decomposed second sensor data, provides at least two different sets of motion reference data in at least two different motion reference data channels. An artifact removal unit determines the vital signal formed from a linear combination of the first sensor data and the motion reference data of at least one two of the motion reference data channels.

It is an object of the invention to provide improved methods and apparatus for monitoring patients.

According to an aspect of the invention, there is provided the computer-implemented method of claim 1.

Thus, a method is provided in which dominant frequencies from multiple different partial reconstructions of measurement data are used to provide an output of a frequency of a periodic physiological process. The inventors have found that this approach allows the frequency to be obtained reliably, particularly when the measurement data is contaminated with low-frequency noise that is potentially close to the frequency of the physiological process of interest. In particular, by comparing different dominant frequencies for consistency it is possible to automatically identify situations in which the measurement data will not yield a reliable estimation of the frequency of the periodic physiological process. Output of an estimation using that measurement data can thus be discarded and the method can move on to consider a subsequent segment of measurement data. The number of invalid estimations output in a given measurement period (containing multiple units of the measurement data) can thus be reduced.

In some embodiments, the sensor system comprises one or more accelerometers. The inventors have found that the methodology is particularly beneficial in this context, due to the vulnerability of accelerometers to contamination by low frequency noise, caused for example by movement of a subject being monitored that is not related to the physiological process of interest. The low frequency noise can comprise low frequency edges in the spectra of measurement data, which can be difficult to tell apart from a signal of interest (e.g. RR).

In some embodiments, the frequency of the periodic physiological process comprises a respiration rate (RR). The inventors have found that the methodology is particularly beneficial in this context, particularly where accelerometers are used to provide the measurement data. This is because typical RR frequencies are close or overlap with common low frequency noise components observed when accelerometers are attached to subjects.

The method allows reliable measurements of RR to be achieved using comfortable wearable devices (in contrast to the alternative techniques mentioned in the introductory part of the description, which achieve significantly lower patient compliance). The methodology can be implemented in a computationally efficient manner, which makes it possible for the wearable devices and/or computers with which the wearable devices communicate, to be implemented with minimal computational resources. Sensitivity is high enough to allow accelerometers to be positioned in a variety of locations without compromising the ability to measure RR reliably. This provides greater flexibility for creating wearable devices, facilitates patient comfort and allows accelerometer-based measurements of RR to be made even where it is not possible to position accelerometers on the chest (for example). The method works well for example where accelerometers are worn comfortably on the arm.

The study described below demonstrates the above advantages using real data collected during monitoring of patients following discharge from intensive care units (ICUs). The results of estimated respiratory rate from two different signal modalities (PPG and accelerations) in a continuous long period of time are shown to be in close agreement for many segments of measurement data across patients, despite the fact that the raw signals are severely affected by various motion interferences, sensor fault, and detachment.

According to an alternative aspect of the invention, there is provided the apparatus for monitoring a human or animal subject of claim 15.

Embodiments of the invention will now be described, by way of example only, with reference to the accompanying drawings in which corresponding reference symbols indicate corresponding parts, and in which:
Figure 1 schematically depicts a method of monitoring a subject according to an embodiment;
Figure 2 schematically depicts an apparatus for monitoring a subject;
Figure 3 depicts data flow through functional modules for implementing methods according to embodiments;
Figure 4 is a graph depicting a spectrum obtained by applying FFT to measurement data from subject #27 to which low-pass filtering has been applied;
Figure 5 is a graph depicting a spectrum obtained by applying FFT to measurement data from subject #27 to which ALE has been applied;
Figure 6 depicts graphs depicting spectra obtained by applying FFT to first and second partial reconstructed versions of measurement data from subject #27 to which ALE has been applied;
Figure 7 is a graph depicting a spectrum obtained by applying FFT to first example measurement data from subject #27 to which low-pass filtering has been applied;
Figure 8 is a graph depicting a spectrum obtained by applying FFT to the first example measurement data from subject #27 to which ALE has been applied;
Figure 9 depicts graphs depicting spectra obtained by applying FFT to first and second partial reconstructed versions of the first example measurement data from subject #27 to which ALE has been applied;
Figure 10 is a graph depicting a spectrum obtained by applying FFT to second example measurement data from subject #27 to which low-pass filtering has been applied;
Figure 11 is a graph depicting a spectrum obtained by applying FFT to the second example measurement data from subject #27 to which ALE has been applied;
Figure 12 depicts graphs depicting spectra obtained by applying FFT to first and second partial reconstructed versions of the second example measurement data from subject #27 to which ALE has been applied;
Figure 13 is a graph depicting a spectrum obtained by applying FFT to third example measurement data from subject #27 to which low-pass filtering has been applied;
Figure 14 is a graph depicting a spectrum obtained by applying FFT to the third example measurement data from subject #27 to which ALE has been applied;
Figure 15 depicts graphs depicting spectra obtained by applying FFT to first and second partial reconstructed versions of the third example measurement data from subject #27 to which ALE has been applied;
Figure 16 is a graph depicting a spectrum obtained by applying FFT to fourth example measurement data from subject #27 to which low-pass filtering has been applied;
Figure 17 is a graph depicting a spectrum obtained by applying FFT to the fourth example measurement data from subject #27 to which ALE has been applied;
Figure 18 depicts graphs depicting spectra obtained by applying FFT to first and second partial reconstructed versions of the fourth example measurement data from subject #27 to which ALE has been applied;
Figures 19(a)-(c) depict the results of measuring respiration rate using a series of segments of measurement data from accelerometers applied to subject #27; and
Figure 20 is a graph comparing measurements of RR using a method of an embodiment with measurements of RR obtained from PPG signals and measurements of RR recorded manually by nurses in an average of every 6 hours, for subject #18.

Methods of the present disclosure are computer-implemented. Each step of the disclosed methods may therefore be performed by a computer. The computer may comprise various combinations of computer hardware, including for example CPUs, RAM, SSDs, motherboards, network connections, firmware, software, and/or other elements known in the art that allow the computer hardware to perform the required computing operations. The required computing operations may be defined by one or more computer programs. The one or more computer programs may be provided in the form of media, optionally non-transitory media, storing computer readable instructions. When the computer readable instructions are read by the computer, the computer performs the required method steps. The computer may consist of a self-contained unit, such as a general-purpose desktop computer, laptop, tablet, mobile telephone, smart device (e.g. smart TV), etc. Alternatively, the computer may consist of a distributed computing system having plural different computers connected to each other via a network such as the internet or an intranet.

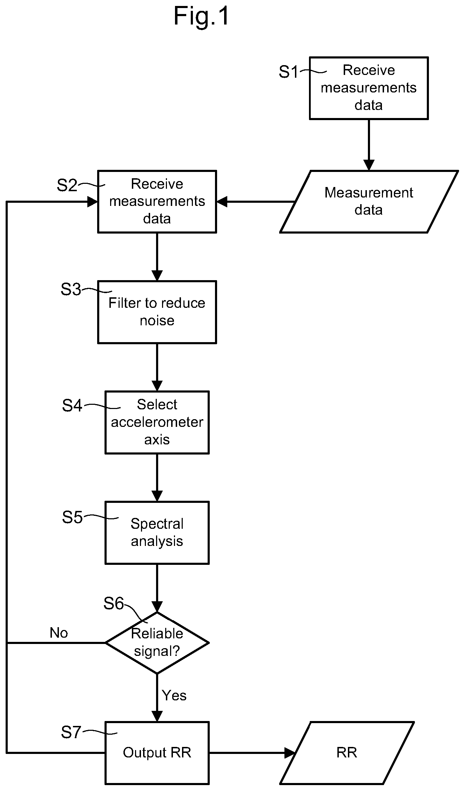

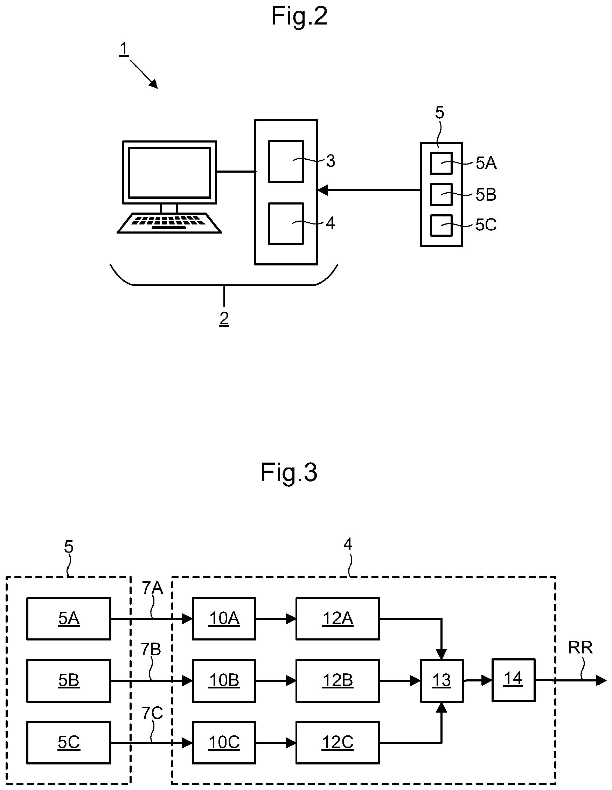

Figure 1 schematically depicts a framework for methods of monitoring a human or animal subject according to embodiments of the disclosure. The methods may be performed by an apparatus 1 for monitoring a human or animal subject as depicted in Figure 2. The terms "human or animal subject" or "subject" may be used interchangeably with the term "patient" in the following description.

The method comprises a step S1 of performing physiological measurements on a subject to obtain measurement data. The physiological measurements may be performed using a sensor system 5 as depicted schematically in Figure 2. In an embodiment, the sensor system 5 comprises a plurality of sensors, each sensor capable of obtaining measurement data using a different measurement mode. As depicted schematically in Figure 3, the measurement data may thus comprise plural channels 7A-C, each channel 7A-7C representing measurements made using a different measurement mode. In embodiments described in detail below, the different measurement modes respectively comprise measurements of acceleration relative to a different respective axis. The sensor system 5 may thus comprise plural accelerometers, for example three accelerometers 5A-C. Each accelerometer 5A-C may be configured to detect acceleration relative to a respective one of three different orthogonal axes. In examples described below, these axes are referred to respectively as "lateral", "vertical" and "longitudinal".

In step S2, measurement data obtained by performing the physiological measurements in step S1 is received by a data receiving unit 3. The data receiving unit 3 may form part of a computing system 2 (e.g. laptop computer, desktop computer, smart device, wearable smart device, etc.). The computing system 2 may further comprise a data processing unit 4 configured to carry out steps of the method. Figure 3 depicts functional modules that may be implemented by the computing system 2 when performing the method.

In step S3, the measurement data is filtered to at least partially remove noise from the measurement data. The filtering may comprise applying a band-pass filter or low-pass filter. Alternatively, as described in further detail below, an adaptive line enhancer (ALE) may be applied to the measurement data. As depicted in Figure 3, the filtering may be applied separately to each channel 7A-C of the measurement data via respective filter modules 10A-C.

In step S4, a signal-to-noise ratio of the filtered measurement data in each channel 7A-C is obtained. In Figure 3, the signal-to-noise ratios are determined by respective signal-to-noise ratio modules 12A-C. The determined signal-to-noise ratios are used (in selection module 13 of Figure 3) to select one of the channels 7A-C to use in subsequent steps to provide the output representing the frequency of the periodic physiological process. In an embodiment, the channel 7A-C with the highest signal-to-noise ratio is selected for subsequent processing. The method may be implemented in such a way as to repeatedly output a measurement of the frequency of the periodic physiological process. This may be achieved for example by processing windows of time series that are centred at different time stamps (each window providing a unit of the measurement data received in step S2 and processed in subsequent steps). The windows may overlap with each other or may not overlap with each other. The windows may be referred to as segments. The selection of the best channel 7A-C may be different for different windows. Thus, the selection of the best channel 7A-C may be dynamically updated in time, switching continuously between different channels for different windows (units of measurement data) according to whichever has the highest signal-to-noise ratio at the time.

In step S5, spectral analysis is performed to extract a frequency of a periodic physiological process of interest. In some embodiments of the present disclosure, the frequency of the periodic physiological process comprises a respiration rate (RR). Further details about the spectral analysis are given below.

In step S6, a determination is made based on the spectral analysis of step S5 about whether the received measurement data is such as to allow a reliable estimate of the frequency of the periodic physiological process to be obtained. If yes, the method continues to step S7, an output is provided representing the extracted frequency of the periodic physiological process for the received measurement data, and the method loops back to step S2 to receive a next unit of measurement data to be processed. If no, the method loops straight back to step S2 to receive a next unit of measurement data to be processed, without providing any output. This methodology thus advantageously reduces the extent to which unreliable readings of the frequency of the periodic physiological process are output.

### Filtering - Adaptive Line Enhancer (ALE)

As mentioned above, in some embodiments an adaptive line enhancer (ALE) is used to at least partially remove noise from measurement data. This process is now described in further detail.

The ALE, introduced in [8], has been used in many applications [9] for separation of a low level sinusoid or a narrow-band oscillation from broad-band noise. The core concept of the ALE is based on linear prediction where the nearly-periodic signal is believed to be perfectly predicted based on the past samples while a non-periodic signal cannot be predicted. A delayed version of the input signal is used as a reference signal into a least-mean-square (LMS) adaptive filter and the desired signal is considered as the original signal. An error signal of the LMS filter provides an estimate of the noise in the input signal and the output signal corresponds to the desired separated signal (i.e. separated from the noise). In specific examples of the present disclosure where it is desired to estimate RR using acceleration signals, the ALE can be applied to each accelerometer axis separately. The ratio of the power of signal to the power of noise provides a signal-to-noise ratio (SNR). The SNR can be used, as described above, to select an accelerometer axis to use in subsequent processing steps (e.g. an axis with the highest SNR is selected).

### Spectral analysis - SSA

After selection of the desired accelerometer axis, Fast Fourier Transform (FFT) could be applied to estimate the highest peak amplitude of the spectrum of ALE signal output in the range of 0.05 Hz to 1 Hz. Then, a frequency with maximum peak amplitude could be obtained, which could correspond to the frequency of the periodic physiological process being monitored (e.g. respiration rate). However, it has been found that the frequency obtained in this way does not accurately reflect the underlying frequency of the periodic process in all situations. For some segments (units) of measurement data, multiple local maximum peaks in the spectral domain are observed, which makes discrimination of the physiological frequency difficult. Embodiments of the present disclosure use the following spectral analysis in step S5 of the method to improve reliability.

In some embodiments, the spectral analysis of step S5 comprises processing the measurement data to represent the measurement data as a mathematical expansion. The measurement data may thus be transformed by the processing of the measurement data, the transformation resulting in the measurement data being represented as a mathematical expansion. The mathematical expansion comprises a plurality of expansion components and a corresponding plurality of expansion coefficients. Each expansion coefficient represents the respective strength of each expansion component in the mathematical expansion. Thus, expansion components which contribute to the measurement data to a large extent will have a large expansion coefficient and expansion components which contribute to the measurement data to a smaller extent will have smaller expansion coefficients. In some embodiments, each expansion component comprises an eigenvector and each expansion coefficient comprises an eigenvalue. In some embodiments, the eigenvectors and eigenvalues are determined using singular spectrum analysis (SSA)

. It has been shown in [11] that after applying SSA into acceleration signals and using the resulting eigenvectors and their corresponding eigenvalues, it is possible to extract the trend of the data or the lowest possible dominant frequency (considering the largest eigenvalues) in the signals.

The inventors have found that by performing partial reconstructions of the measurement data using different combinations of the eigenvectors mentioned above, it is possible to extract the frequency of the physiological process being monitored more accurately and/or identify segments of measurement data from which it is not possible to obtain a reliable estimate of the frequency of the physiological process.

In an embodiment, a first partial reconstruction of the measurement data is formed using a first subset of the expansion components (e.g. eigenvectors) and corresponding expansion coefficients (e.g. eigenvalues). A second partial reconstruction of the measurement data is formed using a second subset of the expansion components (e.g. eigenvectors) and corresponding expansion coefficients (e.g. eigenvalues). The second subset is different from the first subset.

A first spectral analysis (e.g. using a fast Fourier transform, FFT) is then performed on the first partial reconstruction to determine a first dominant frequency. The first dominant frequency represents a frequency component of largest amplitude in the first partial reconstruction.

A second spectral analysis (e.g. using a fast Fourier transform, FFT) is performed on the second partial reconstruction to determine a second dominant frequency. The second dominant frequency represents a frequency component of largest amplitude in the second partial reconstruction.

The first and second partial reconstructions may be referred to respectively as sub-band 1 and sub-band2. The provision of an output representing a frequency of a periodic physiological process of step S7 can be performed based on either or both of the first dominant frequency and the second dominant frequency. In a case where the measurement data is able to yield a reliable value for the physiological frequency of interest, the first dominant frequency and the second dominant frequency should both correspond closely to the physiological frequency (and not some other artefactual frequency present in the measurement data). The output may be obtained for example by taking an average of the first dominant frequency and the second dominant frequency, or simply by taking one or the other of the first dominant frequency and the second dominant frequency (as they are both similar).

The inventors have found that in cases where the measurement data is unable to yield a reliable value for the physiological frequency of interest, for example because there are several spectral peaks in the frequency range of interest, the situation can be detected automatically and reliably by detecting when the first dominant frequency and the second dominant frequency differ from each other to a significant extent (e.g. by more than a predetermined threshold amount). Based on this recognition, according to the invention, a degree of similarity between the first dominant frequency and the second dominant frequency is determined and the output representing the frequency of the periodic physiological process is selectively provided (i.e. provided or not provided) in step S7 depending on the determined degree of similarity. For example, the output is provided if the degree of similarity is determined to be higher than a predetermined threshold.

In some embodiments, the first subset of expansion components (corresponding to sub-bandl) comprises the *N* components respectively having the *N* largest expansion coefficients. *N* may satisfy the following inequality: 2 ≤ *N* ≤ 5. In an embodiment, *N* = 3. In an embodiment, the second subset of expansion components (corresponding to sub-band2) does not comprise the expansion component having the largest expansion coefficient. In an embodiment, the second subset of expansion components comprises the expansion components corresponding to the *N* largest expansion coefficients except the largest expansion coefficient. In an embodiment, the second subset of expansion components comprises the 2^{nd} and 3^{rd} largest.

### Detailed Examples

In the detailed examples described below, the spectral analysis of step S5 was performed on measurement data to which ALE had been applied in step S3 and to an accelerometer axis selected using the ALE output in step S4 to decompose the measurement data into corresponding eigenvectors and eigenvalues. The eigenvectors were sorted in descending order of associated eigenvalues and grouped using a set of indices as {1,2,3} (denoting eigenvectors related to first, second, and third largest eigenvalues) and {2,3} (denoting eigenvectors related to the second and third largest eigenvalues). After grouping the eigenvectors into these two sub-bands (sub-bandl for {1,2,3} indices and sub-band2 for {2,3} indices), a final elementary matrix was formed by summation of all elementary matrices in each group. Diagonal averaging was applied to the final elementary matrix and a resulting signal formed for each group. For each sub-band, one narrowband signal was reconstructed (an example of the partial reconstruction referred to above). An FFT algorithm was then applied to each narrowband signal to estimate and compare the frequency spectrum for the two selected sub-bands.

The FFT was applied to each reconstructed signal corresponding to the sub-bands and the frequency with maximum peak amplitude was considered as a potential respiratory component. Then, for each sub-band, the RR was calculated as breaths per minute (based on the maximum peak). If the absolute difference in estimated RR from the two sub-bands was less than 4 bpm (an example of the predetermined threshold mentioned above), an average RR was used as the final estimated RR, otherwise, it was determined that a reliable RR could not be obtained from the measurement data (the "no" loop in Figure 1). The threshold of 4 bpm was found appropriate for the particular dataset being considered. It is expected that a lower threshold could be used by increasing the frequency resolution.

### Dataset for Detailed Examples

Waveform data were collected from voluntary participants in the Post Intensive Care Risk Alerting and Monitoring (PICRAM) - II trial. This waveform data was collected using continuous monitoring equipment provided by Hidalgo Ltd. with an attached transmittance photoplethysmograph sensor provided by Nonin Medical Inc. The data was collected at both the John Radcliffe and the Reading Berkshire hospital. In this research only the data collected at Oxford John Radcliffe was used for analysis.

Time-stamped observations for patients admitted to the ward at the John Radcliffe hospital were recorded within the experiment. The data corresponds to admissions between 2013 and 2014. Sampling frequencies were 256 Hz for an electrocardiogram (ECG), 75 Hz for a PPG, and 25 Hz for an accelerometer.

Ten patients (five male, five female) were selected from a larger dataset. These selected patients' age range was from 45 to 77 years old at the time of discharge from the ICU. The select patients' mean age was calculated as 63.7 years old. The length of their stay at the ICU ranged from 1 day to 15 days. The hospital length of stay for these patients ranged from 7 days to 47 days. All patients were white except one with black/British black ethnicity.

### Results for Detailed Examples

For estimation of RR from accelerometer signals, the ALE was applied to each axis of the accelerometer signal to separate the oscillatory and noise component of the accelerometer axes. A delay (*δ*) of 10 samples was used to generate the reference signal for the adaptive filter. The normalised least mean square (NLMS) adaptive filter with an order of (M = 20), and an offset of (N = 50) was applied. Units of measurement data were obtained by forming 64-second windowed data segments and discarding the first 16 seconds of each data segment. Each resulting 48-seconds long data segment corresponded to one unit of measurement data to be processed. A reduced window size of 48-seconds promotes convergence of the NLMS. The ALE separates the noise and signal component of each accelerometer axis.

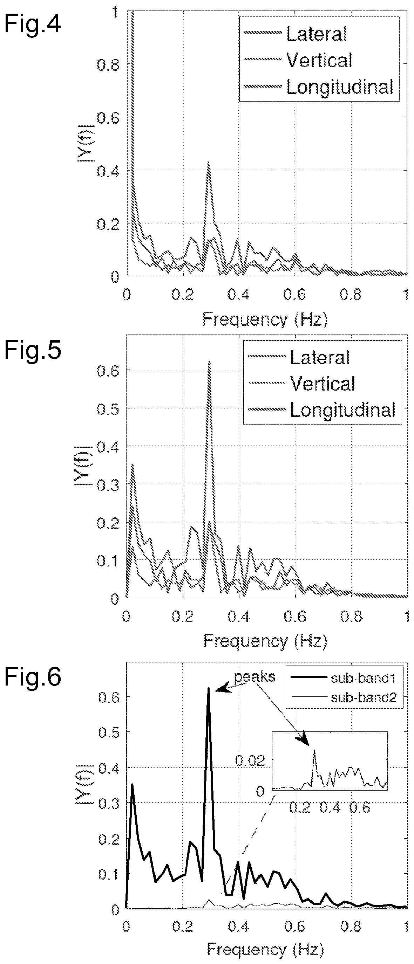

Figures 4-6 compare the processed signal for each accelerometer axis in the spectral domain using FFT for an example unit of measurement data. The SNR as the power of signal to the power of noise was calculated as 0.19, 0.11 and 0.32 for axis 1 (lateral), 2 (vertical), and 3 (longitudinal) respectively. The highest SNR corresponded to axis 3 (longitudinal axis). This axis was selected for further analysis to estimate RR.

Figure 4 shows the result of applying an FFT to the measurement data for each accelerometer axis after a low-pass filter is applied in step S3 of the method. The low-pass filtered spectrum shows a similar maximum peak (considering frequencies more than 0.1Hz) for each of the three accelerometer axes.

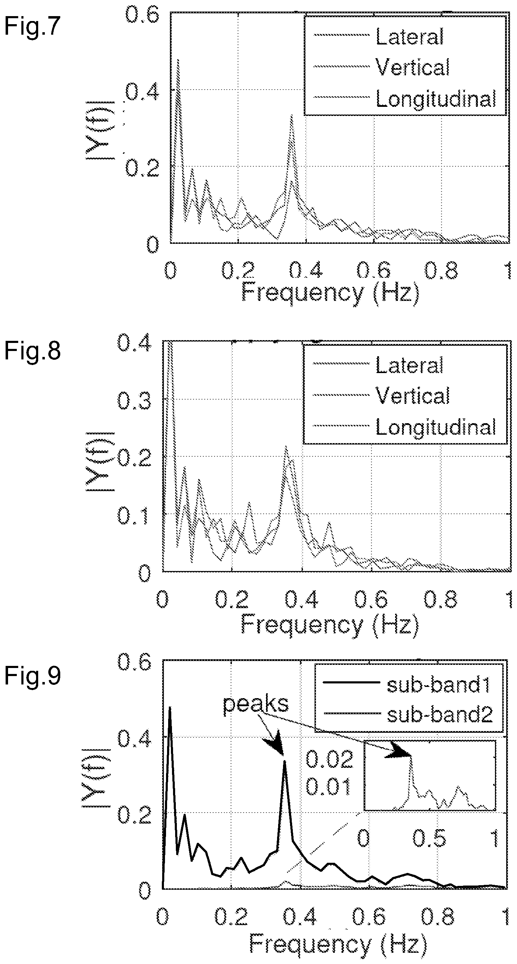

Figure 5 shows the result of applying the FFT to the measurement data for each accelerometer axis after an ALE is applied in step S3 of the method. The resulting spectra are visibly enhanced relative to Figure 4. The selected accelerometer axis with the largest SNR (third axis for this segment) was further subjected to the spectral sub-band decomposition procedure using SSA, with the results being shown in Figure 6. This procedure improves analysis of the acceleration signals, particularly where, for example, a sharp change of amplitudes in the acceleration signal creates a low frequency in the spectral domain that is not related to the respiratory component. As explained above, two new signals are reconstructed in sub-band1 and sub-band2. Then, FFT is applied to output an estimated RR where the difference of estimated RR in the two specified sub-bands is less than 4bpm. For patient #27, at Jun.06.2013 13:50, a manual observation of RR as 19 bpm was recorded. In the midnight of Jun.07.2013, after about 10 hours and 10 minutes, a manual observation of RR as 22 bpm was recorded. In this time interval, the maximum, minimum and mean recorded RR was obtained as 22 bpm, 18 bpm and 19.92 bpm. The selection of this patient is based on low variability of the manual observations to demonstrate cases where applying FFT alone to the accelerometer axes is not effective. In addition, applying SSA sub-band decomposition to select an appropriate frequency band to estimate RR is necessary. A wrongly selected frequency sub-band could lead to major errors of 10 bpm or more in estimation of RR. Figure 6 shows the result of applying the FFT to each sub-band using SSA, where the first sub-band related to grouping {1,2,3} eigenvectors has greater peak amplitude comparing to the second sub-band {2,3}. In both sub-bands the maximum spectral peaks are in close agreement. For this segment (unit) of measurement data, the estimated RR was obtained as 17.62 bpm at June.06.2013 14:03:23. The closest manual observation was recorded as 19 bpm at June.06.2013 13:50:00. In Figures 7-18, four examples from subject #27 are provided to further demonstrate that applying both ALE and SSA sub-band decomposition assists with providing a reliable estimation of RR. For each example, three graphs corresponding respectively to the graphs of Figures 4-6 are provided.

*Example 1:* Figures 7-9 show that all three accelerometer axes provide measurement data containing a dominant low frequency peak around a frequency of ≈ 0.35Hz (≈ 21 bpm), both when processing is based on low pass filtering in step S3 (Figure 7) and when processing is based on ALE in step S3 (Figure 8). The segmented window corresponding to the measurement data for Example 1 starts at Jun.06.2013 19:30:28 where a manual RR of 22 was recorded at Jun.06.2013 19:30:00. The estimated RR using the proposed method was calculated as 21.39 bpm. The maximum spectral peak is in a good agreement to the recorded manual observation.

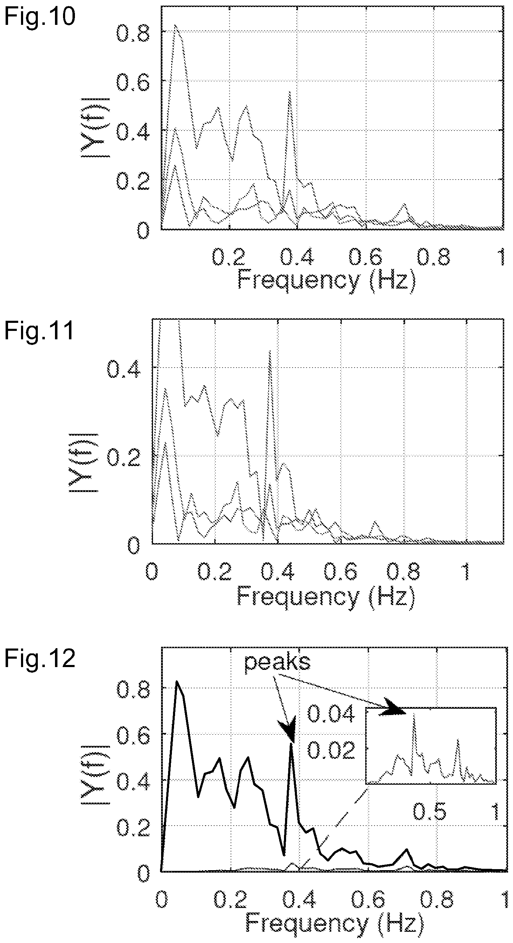

*Example 2:* Figures 10-12 show results for an example in which two of the accelerometer axes demonstrate a dominant spectral peak around a frequency of 0.38Hz (22.8 bpm), while the other axis shows a maximum peak around 0.3Hz (15 bpm). This example shows that selection of the best accelerometer axis is advantageous. The ALE can be used to find the best axis using SNR information, as described above.

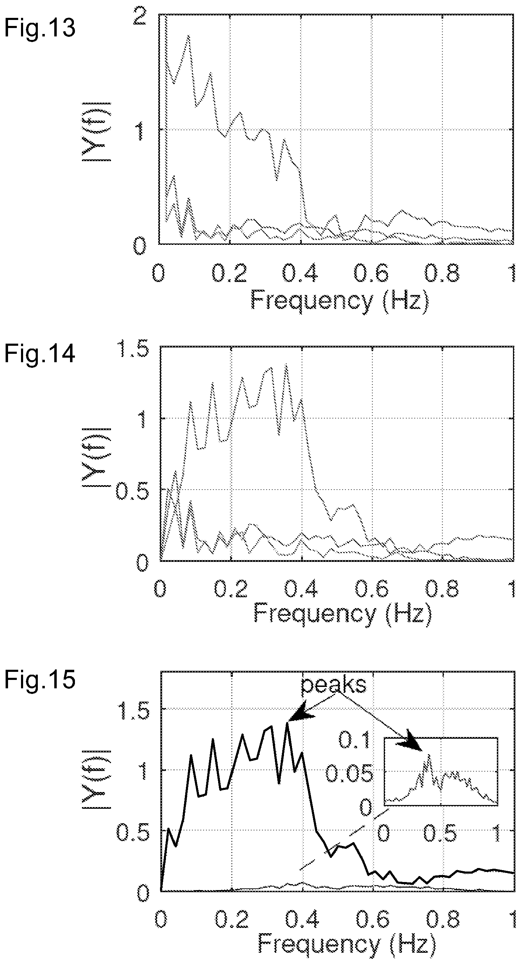

*Example 3:* Figures 13-15 show results for an example in which there is a strong low frequency component which has affected the results after low-pass filtering. This example shows that FFT applied to raw acceleration produces a maximum peak in frequencies of less than 0.2 Hz (Figure 13 - relating to a respiratory rate of about 12 bpm). A band-pass filter with lower and upper cut-off frequencies as 0.05 Hz and 1 Hz, can also been applied to raw acceleration where the maximum peak happens before 0.2 Hz. The output of the ALE producing the spectrum of Figure 14 yields SNRs of 0.31, 0.17, 1.46 for axis 1 (lateral), 2 (vertical), and 3 (longitudinal) respectively. The FFT applied to the third axis (which has the highest SNR) produced a spectral peak around 0.4Hz after applying FFT to the signal ALE output (see Figure 14). The sub-band decomposition of the ALE signal output of the third axis is shown in Figure 15, where the largest peaks in both sub-bands are in close agreement, thereby indicating that an RR estimate based on the largest peaks is reliable. The average of these peaks was used to estimate the final RR. For this segment of measurement data, the estimated RR was 23.91 bpm at Jun.06.2013 19:33:36, where the closest bpm observed manually was 22 bpm at Jun.06.2013 19:30:00. For this example, only a band-pass filter with cut-off frequencies of [0.2Hz-0.5Hz] could derive the frequency spectrum with a peak around 0.4 Hz, while our method is able to find a good match in filtered signals in two sub-bands without using such a narrow band-pass filter, which would not be appropriate when seeking to detect transitions between normal and abnormal states of the subject.

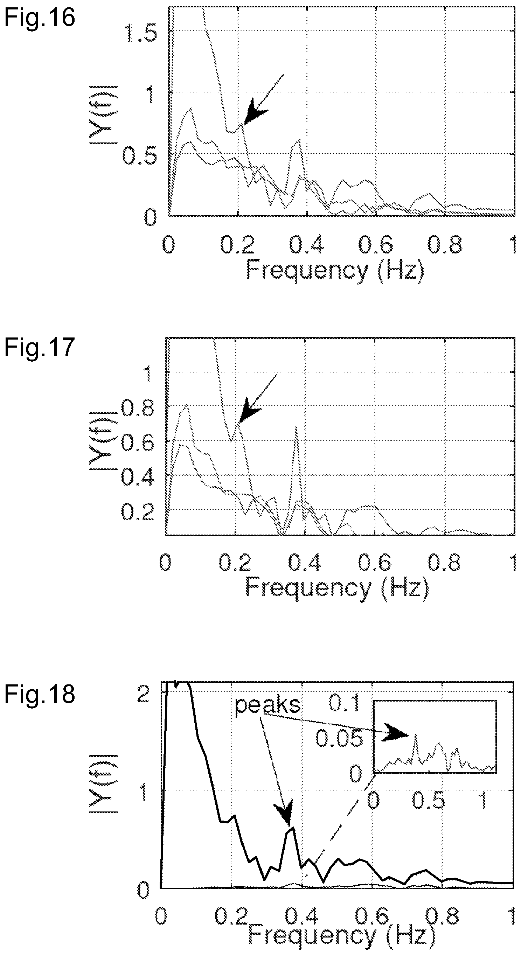

*Example 4:* Figures 16-18 show results for an example in which a low frequency edge is present in the measurement data, which can produce uncertainty in the RR estimation. For example, an edge frequency of about ≈ 0.2Hz (≈ 12 bpm) will be picked after applying low-pass filtering and also the SSA algorithm - sub-bandl - as the maximal spectral peak (see arrows), while there is also a dominant frequency at ≈ 0.38Hz (≈ 22.8 bpm), which appeared in sub-band2 as well. Based on prior knowledge, investigating manual observations and matching to PPG-based estimates, the frequency of about 0.38 (22.8 bpm) is the actual RR related frequency. For this example, our method thus failed to select the correct peak corresponding to the RR. However, the lack of agreement in the frequency domain between the two reconstructed sub-band signals identifies the problem. The uncertainty in the estimation of RR is identified and no RR is returned by the proposed algorithm. The peak corresponding to 12 bpm is most probably related to a frequency edge and very likely a result of an induced transient motion. Therefore, it is invalid.

Uniform acceleration signals are expected to provide clear spectra. By detailed examination of the acceleration signals, it is found that many types of motions, especially transient motion interferences not related to respiration, provide low frequency edges or artefacts in the accelerometer spectra. Examples of spectra containing such low frequency artefacts are shown in Examples 3 and 4 and it is described how our method provides sufficient sensitivity to extract the correct RR values and/or reliably recognise when a reading is probably invalid.

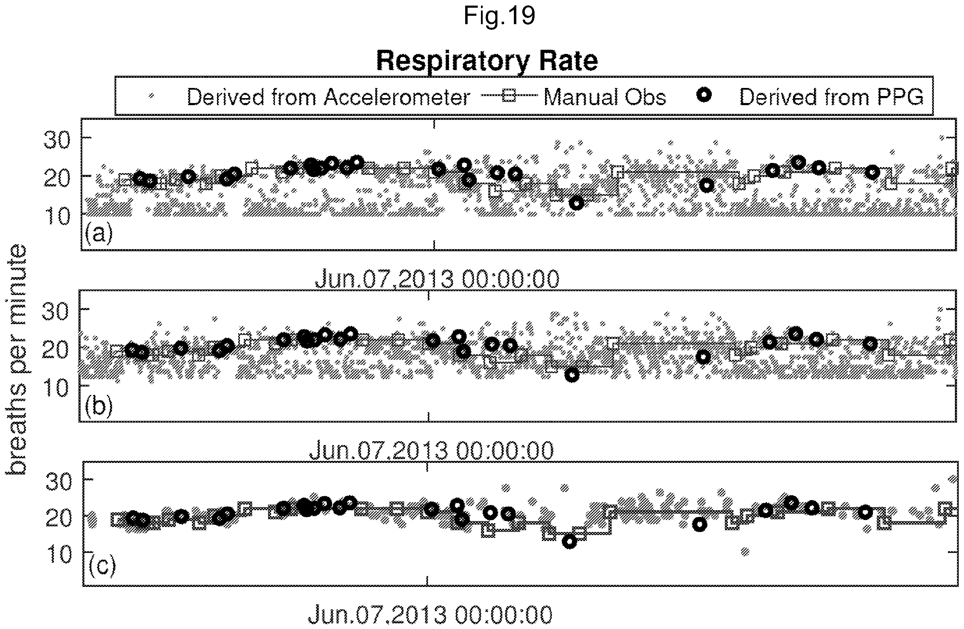

Figures 19(a)-(c) depict the results of measuring respiration rate using a series of segments (units) of measurement data from accelerometers applied to subject #27. Figure 19(a) depicts the result of applying an FFT to segments of measurement data to which a band pass filter in the range of [0.1Hz-1Hz] has been applied (compared to manual observations and measurements using PPG). Figure 19(b) depicts the result of applying an FFT to segments of measurement data to which a band pass filter in the range of [0.2Hz-1Hz] has been applied (compared to manual observations and measurements using PPG). For both Figures 19(a) and 19(b), a maximum spectral peak in the low-frequency range of [0.08Hz-0.5Hz] among all the axes after applying FFT was used to estimate RR.

Figure 19(c) depicts the result of using a method according to an embodiment to determine RR. The method included use of an ALE in step S3 and SSA in step S5. It is evident from Figures 19(a)-(c) that the estimated RR does vary continuously from 10 to 20. By detailed investigation of segments of the measurement data, it was found that transient motion induced quantities affect the signal spectrum in the low frequency domain, creating (invalid) RR readings of ≈ 10 bpm related to a picked frequency edge. By testing several segments of measurement data producing an RR of ≈ 10 bpm and slightly reducing or shifting windowed segments of measurement data to include only uniform accelerations, RRs of around 20 bpm were re-calculated. This confirmed continuous change of breathing rate from ≈ 20 bpm to ≈ 10 bpm is mostly invalid. As Figure 19(c) demonstrates, our method minimizes the effects of low frequency artefacts in respiration derived spectra by using the SSA method which attempts to find a shared low frequency component in the reconstructed signals of two sub-bands. Good agreement is seen between manual observation, accelerometer and PPG-based estimates for subject #27, highlighting the advantages of our method.

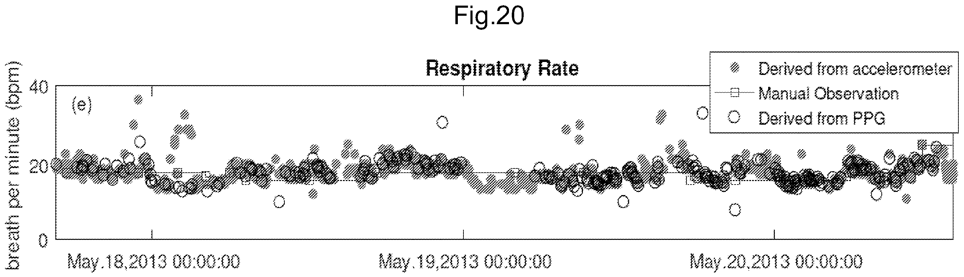

For the patients selected in this study, measurements of RR using a method of an embodiment were compared with measurements of RR obtained from PPG signals and measurements of RR recorded manually by nurses in an average of every 6 hours. The results of such a comparison are shown in Figure 20 for subject #18. This subject shows continuous recorded signals for more than 2 days. The accelerometry-based estimations of RR overlap strongly with both the PPG-based estimations of RR and the manual observations in the plot of Figure 20, demonstrating strong agreement between RR estimates for most parts of the recorded signals.

In Table I, for 10 selected patients, detailed statistics are provided. Based on this table, the number of PPG segments to estimate RR is shown in the second column for each subject. These segments are converted to their time duration in terms of the number of hours of recorded PPG data. Then, the number of segments with a PPG Signal Quality Index (SQI), indicated a quality of the signal for the segment in question, of greater than or equal to 0.85 is provided in the third column. The percentage of segments produced RR estimates based on smart fusion of PPG data (agreement of auto-regression, AR, spectrum in two modulations) are shown for each subject. For accelerometer-based estimation of RR, the initial number of segments of measurement data and the corresponding time duration in terms of the hours of data are provided. Also the percentage of obtained segments to estimate RR based on the spectral analysis approach involving agreement of sub-bands (as discussed above) is provided.

The time-stamps have been used to compare the errors of pairwise estimated RRs where both PPG and accelerometer-based RR estimates are available. The mean absolute error (MAE) is shown in the last column of Table I. As it can be seen from Table I, all the resulted MAEs are less than 2.60 bpm. It should be noted that the sample size to calculate the MAE is not the same for all the subjects. Meanwhile, very good MAEs have been achieved for most subjects (less than 2 bpm). The accelerometers have been able to produce RR estimates for about 70 hours of the continuous data while this has been recorded for about 27 hours of PPG data. Although after data fusion, for error measurements, the number of segments are reduced to consider highly reliable RR outputs of the algorithms.

**TABLE 1**

| COMPARISON OF PPG AND ACCELEROMETER RR ESTIMATES | | | | | | |
|---|---|---|---|---|---|---|
| | PPG | | | Accelerometer | | Difference |
| patient ID | segments No. (hours) | segment No. SQI ≥ 0.85 | ratio (%) of smart fusion | segments No. (hours) | ratio (%) of spectral fusion | MAE (bpm) |
| 18 | 1543 (13.72) | 1281 | 20.69 | 3989 (70.92) | 28.48 | 1.34 |
| 16 | 2403 (21.36) | 2018 | 12.00 | 1397 (24.84) | 1246 | 1.81 |
| 34 | 2699 (23.10) | 812 | 23.40 | 1450 (25.78) | 18.00 | 1.24 |
| 43 | 3121 (27.75) | 2930 | 17.62 | 3993 (70.99) | 12.28 | 1.56 |
| 45 | 3007 (26.73) | 1923 | 15.61 | 1540 (27.38) | 19.81 | 1.52 |
| 36 | 502 (4.47) | 351 | 12.54 | 1488 (26.46) | 18.08 | 1.84 |
| 13 | 1514 (13.46) | 946 | 11.89 | 3976 (70.69) | 6.19 | 2.56 |
| 15 | 1519 (13.51) | 200 | 22.00 | 3925 (69.78) | 9.69 | 0.90 |
| 23 | 533 (4.74) | 404 | 10.15 | 1375 (24.45) | 25.10 | 1.73 |
| 27 | 637 (5.67) | 507 | 5.13 | 1655 (29.43) | 16.02 | 1.86 |

### REFERENCES

[1] P. Jiang and R. Zhu, "Dual tri-axis accelerometers for monitoring physiological parameters of human body in sleep," IEEE Sensors, 2016.
[2] M. Haescher, D. J. C. Matthies, J. Trimpop, and B. Urban, "A study on measuring heart- and respiration-rate via wrist-worn accelerometer-based seismocardiography (SCG) in comparison to commonly applied technologies," Proceedings of the 2nd international Workshop on Sensor-based Activity Recognition and Interaction, 2015.
[3] D. S. Morillo, L. F. C. F. J. L. Rojas Ojeda, and A. Jimenez, "An accelerometer-based device for sleep apnea screening," IEEE Trans. Inf. Technol. Biomed., vol. 14, pp. 491 - 499, 2010.
[4] Z. Zhang and G. Z. Yang, "Monitoring cardio-respiratory and posture movements during sleep: What can be achieved by a single motion sensor," 2015 IEEE 12th International Conference on Wearable and Implantable Body Sensor Networks (BSN), 2015.
[5] S. P. Jeelani, P. Maniyar, J. Joseph and M. Sivaprakasam, "Accelerometer based system for continuous respiratory rate monitoring," IEEE International Symposium on Medical Measurements and Applications (MeMeA), pp. 171-176, 2017.
[6] A. Bates, M. J. Ling, J. Mann and D. K. Arvind, "Respiratory Rate and Flow Waveform Estimation from Tri-axial Accelerometer Data," International Conference on Body Sensor Networks, Singapore,, pp. 144-150,2010.
[7] A. M. Chan, N. Ferdosi and R. Narasimhan, "Ambulatory respiratory rate detection using ECG and a triaxial accelerometer," Conf Proc IEEE Eng Med Biol Soc., pp. 4058-4061, 2013.
[8] B. Widrow, J. Glover, J. McCool, J. Kaunitz, C. Williams, R. Hern, J. Zeidler, E. Dong, and R. Goodlin, "Adaptive noise canceling principles and applications," Proceedings of IEEE, vol. 63, pp. 1692 1716, 1975.
[9] R. M. Ramli, A. O. A. Noor, and S. A. Samad, "A review of adaptive line enhancers for noise cancellation," Australian Journal of Basic and Applied Sciences, vol. 6, no. 6, pp. 337-352, 2012.
[10] N. G. V. Nekrutkin and A. Zhigljavsky, Analysis of Timeseries Structure: SSA and Related Techniques. Chapman and Hall/ CRC, 2001.
D. Jarchi, C. Wong, R. M. Kwasnicki, B. Heller, G. A. Tew, and G. Z. Yang, "Gait parameter estimation from a miniaturized ear-worn sensor using singular spectrum analysis and longest common subsequence," IEEE Trans. Biomed. Eng., pp. 1261 - 1273, 2014.

## Claims

1. A computer-implemented method of monitoring a human or animal subject, comprising:
(a) receiving measurement data representing a time series of measurements on a subject performed by a sensor system (5);
(b) processing the measurement data to represent the measurement data as a mathematical expansion comprising a plurality of expansion components and a corresponding plurality of expansion coefficients representing the respective strength of each expansion component;
(c1) forming a first partial reconstruction of the measurement data using a first subset of the expansion components and corresponding expansion coefficients;
(c2) forming a second partial reconstruction of the measurement data using a second subset of the expansion components and corresponding expansion coefficients, the second subset being different from the first subset;
(d1) performing a first spectral analysis on the first partial reconstruction to determine a first dominant frequency, the first dominant frequency representing a frequency component of largest amplitude in the first partial reconstruction;
(d2) performing a second spectral analysis on the second partial reconstruction to determine a second dominant frequency, the second dominant frequency representing a frequency component of largest amplitude in the second partial reconstruction; and
(e) providing an output representing a frequency of a periodic physiological process based on either or both of the first dominant frequency and the second dominant frequency, wherein:
the method further comprises determining a degree of similarity between the first dominant frequency and the second dominant frequency; and
the output representing the frequency of the periodic physiological process is selectively provided depending on the determined degree of similarity.

2. The method of claim 1, wherein the sensor system (5) comprises one or more accelerometers.

3. The method of claim 1 or 2, wherein the frequency of the periodic physiological process comprises a respiration rate.

4. The method of any preceding claim, wherein steps (a)-(d2) are repeated for plural units of the measurement data, and step (e) comprises outputting the frequency of the periodic physiological process only for units of the measurement data for which the determined degree of similarity between the first dominant frequency and the second dominant frequency is above a predetermined threshold.

5. The method of any preceding claim, wherein each expansion component comprises an eigenvector and each expansion coefficient comprises an eigenvalue.

6. The method of claim 5, wherein the eigenvectors and eigenvalues are determined using singular spectrum analysis.

7. The method of any preceding claim, wherein the first subset of expansion components comprises the *N* components respectively having the *N* largest expansion coefficients, wherein preferably 2 ≤ *N* ≤ 5.

8. The method of claim 7, wherein the second subset of expansion components does not comprise the expansion component having the largest expansion coefficient.

9. The method of claim 8, wherein the second subset of expansion components comprises the expansion components corresponding to the N largest expansion coefficients except the largest expansion coefficient.

10. The method of any preceding claim, wherein the measurement data is processed to at least partially removing noise from the measurement data in a filtering step prior to the processing to represent the measurement data as a mathematical expansion, preferably wherein the filtering step comprises applying a low-pass filter or an adaptive line enhancer.

11. The method of claim 10, wherein:
the measurement data comprises multiple channels, each channel representing measurements made using a different measurement mode; and
the method comprises determining a signal-to-noise ratio of the measurement data in each channel, after the filtering step, and using the determined signal-to-noise ratio to select one of the channels to use in subsequent steps to provide the output representing the frequency of the periodic physiological process.

12. The method of claim 11, wherein the different measurement modes each comprise measurements of acceleration relative to a different respective axis.

13. A computer program comprising computer-readable instructions that cause a computer to perform the method of any preceding claim.

14. A computer-readable medium storing the computer program of claim 13.

15. An apparatus (1) for monitoring a human or animal subject, comprising:
a data receiving unit (3) configured to receive measurement data representing a time series of measurements on a subject performed by a sensor system (5);
a data processing unit (4) configured to:
process the measurement data to represent the measurement data as a mathematical expansion comprising a plurality of expansion components and a corresponding plurality of expansion coefficients representing the respective strength of each expansion component;
form a first partial reconstruction of the measurement data using a first subset of the expansion components and corresponding expansion coefficients;
form a second partial reconstruction of the measurement data using a second subset of the expansion components and corresponding expansion coefficients, the second subset being different from the first subset;
perform a first spectral analysis on the first partial reconstruction to determine a first dominant frequency, the first dominant frequency representing a frequency component of largest amplitude in the first partial reconstruction;
perform a second spectral analysis on the second partial reconstruction to determine a second dominant frequency, the second dominant frequency representing a frequency component of largest amplitude in the second partial reconstruction; and
provide an output representing a frequency of a periodic physiological process based on either or both of the first dominant frequency and the second dominant frequency, wherein:
the data processing unit (4) is further configured to determine a degree of similarity between the first dominant frequency and the second dominant frequency; and
the data processing unit (4) is further configured to selectively provide the output representing the frequency of the periodic physiological process depending on the determined degree of similarity.

## Patentansprüche

1. Computer-implementiertes Verfahren zur Überwachung eines menschlichen oder tierischen Subjekts, wobei das Verfahren Folgendes umfasst:
(a) Empfangen von Messdaten, die eine Zeitreihe von Messungen an einem Subjekt darstellen, die von einem Sensorsystem (5) durchgeführt werden;
(b) Verarbeiten der Messdaten, um die Messdaten als eine mathematische Expansion darzustellen, die eine Vielzahl von Expansionskomponenten und eine entsprechende Vielzahl von Expansionskoeffizienten umfasst, die die jeweilige Stärke jeder Expansionskomponente darstellen;
(c1) Bilden einer ersten Teilrekonstruktion der Messdaten unter Verwendung einer ersten Teilmenge der Expansionskomponenten und der entsprechenden Expansionskoeffizienten;
(c2) Bilden einer zweiten Teilrekonstruktion der Messdaten unter Verwendung einer zweiten Teilmenge der Expansionskomponenten und der entsprechenden Expansionskoeffizienten, wobei die zweite Teilmenge von der ersten Teilmenge verschieden ist;
(d1) Durchführen einer ersten Spektralanalyse an der ersten Teilrekonstruktion, um eine erste dominante Frequenz zu bestimmen, wobei die erste dominante Frequenz eine Frequenzkomponente mit der größten Amplitude in der ersten Teilrekonstruktion darstellt;
(d2) Durchführen einer zweiten Spektralanalyse an der zweiten Teilrekonstruktion, um eine zweite dominante Frequenz zu bestimmen, wobei die zweite dominante Frequenz eine Frequenzkomponente mit der größten Amplitude in der zweiten Teilrekonstruktion darstellt; und
(e) Bereitstellen einer Ausgabe, die eine Frequenz eines periodischen physiologischen Prozesses darstellt, basierend auf der ersten dominanten Frequenz oder der zweiten dominanten Frequenz oder beidem, wobei:
das Verfahren ferner die Bestimmung eines Ähnlichkeitsgrades zwischen der ersten dominanten Frequenz und der zweiten dominanten Frequenz umfasst; und
die Ausgabe, die die Frequenz des periodischen physiologischen Prozesses darstellt, selektiv in Abhängigkeit von dem bestimmten Ähnlichkeitsgrad bereitgestellt wird.

2. Verfahren nach Anspruch 1, wobei das Sensorsystem (5) einen oder mehrere Beschleunigungsmesser umfasst.

3. Verfahren nach Anspruch 1 oder 2, wobei die Frequenz des periodischen physiologischen Prozesses eine Atmungsrate umfasst.

4. Verfahren nach einem der vorhergehenden Ansprüche, wobei die Schritte (a) bis (d2) für mehrere Einheiten der Messdaten wiederholt werden und Schritt (e) die Ausgabe der Frequenz des periodischen physiologischen Prozesses nur für Einheiten der Messdaten ausgibt, für die der ermittelte Ähnlichkeitsgrad zwischen der ersten dominanten Frequenz und der zweiten dominanten Frequenz über einem vorgegebenen Schwellenwert liegt.

5. Verfahren nach einem der vorhergehenden Ansprüche, wobei jede Expansionskomponente einen Eigenvektor und jeder Expansionskoeffizient einen Eigenwert umfasst.

6. Verfahren nach Anspruch 5, wobei die Eigenvektoren und Eigenwerte unter Verwendung der Singulärspektrumanalyse bestimmt werden.

7. Verfahren nach einem der vorhergehenden Ansprüche, wobei die erste Teilmenge von Expansionskomponenten die *N* Komponenten mit den jeweils *N* größten Expansionskoeffizienten umfasst, wobei vorzugsweise 2 ≥ *N* ≥ *5.*

8. Verfahren nach Anspruch 7, wobei die zweite Teilmenge von Expansionskomponenten nicht die Expansionskomponente mit dem größten Expansionskoeffizienten umfasst.

9. Verfahren nach Anspruch 8, wobei die zweite Teilmenge von Expansionskomponenten die Expansionskomponenten umfasst, die den *N* größten Expansionskoeffizienten mit Ausnahme des größten Expansionskoeffizienten entsprechen.

10. Verfahren nach einem der vorhergehenden Ansprüche, wobei die Messdaten verarbeitet werden, um zumindest teilweise Rauschen aus den Messdaten in einem Filterungsschritt vor der Verarbeitung zu entfernen, um die Messdaten als eine mathematische Expansion darzustellen, wobei der Filterungsschritt vorzugsweise die Anwendung eines Tiefpassfilters oder eines adaptiven Linienverstärkers umfasst.

11. Verfahren nach Anspruch 10, wobei:
die Messdaten mehrere Kanäle umfassen, wobei jeder Kanal Messungen darstellt, die unter Verwendung eines anderen Messmodus durchgeführt wurden; und
das Verfahren das Bestimmen eines Signal-Rausch-Verhältnisses der Messdaten in jedem Kanal nach dem Filterungsschritt und das Verwenden des bestimmten Signal-Rausch-Verhältnisses umfasst, um einen der Kanäle auszuwählen, der in den nachfolgenden Schritten zu verwenden ist, um die Ausgabe bereitzustellen, die die Frequenz des periodischen physiologischen Prozesses darstellt.

12. Verfahren nach Anspruch 11, wobei die verschiedenen Messmodi jeweils Messungen der Beschleunigung relativ zu einer anderen jeweiligen Achse umfassen.

13. Computerprogramm mit computerlesbaren Anweisungen, die einen Computer veranlassen, das Verfahren nach einem der vorhergehenden Ansprüche auszuführen.

14. Computerlesbares Medium, das das Computerprogramm nach Anspruch 13 speichert.

15. Vorrichtung (1) zur Überwachung eines menschlichen oder tierischen Subjekts, wobei die Vorrichtung Folgendes umfasst:
eine Datenempfangseinheit (3), die dafür konfiguriert ist, Messdaten zu empfangen, die eine Zeitreihe von Messungen an einem Subjekt darstellen, die von einem Sensorsystem (5) durchgeführt werden;
eine Datenverarbeitungseinheit (4), die für Folgendes konfiguriert ist:
Verarbeiten der Messdaten, um die Messdaten als eine mathematische Expansion darzustellen, die eine Vielzahl von Expansionskomponenten und eine entsprechende Vielzahl von Expansionskoeffizienten umfasst, die die jeweilige Stärke jeder Expansionskomponente darstellen;
Bilden einer ersten Teilrekonstruktion der Messdaten unter Verwendung einer ersten Teilmenge der Expansionskomponenten und der entsprechenden Expansionskoeffizienten;
Bilden einer zweiten Teilrekonstruktion der Messdaten unter Verwendung einer zweiten Teilmenge der Expansionskomponenten und der entsprechenden Expansionskoeffizienten, wobei die zweite Teilmenge von der ersten Teilmenge verschieden ist;
Durchführen einer ersten Spektralanalyse an der ersten Teilrekonstruktion, um eine erste dominante Frequenz zu bestimmen, wobei die erste dominante Frequenz eine Frequenzkomponente mit der größten Amplitude in der ersten Teilrekonstruktion darstellt;
Durchführen einer zweiten Spektralanalyse an der zweiten Teilrekonstruktion, um eine zweite dominante Frequenz zu bestimmen, wobei die zweite dominante Frequenz eine Frequenzkomponente mit der größten Amplitude in der zweiten Teilrekonstruktion darstellt; und
Bereitstellen einer Ausgabe, die eine Frequenz eines periodischen physiologischen Prozesses darstellt, basierend auf der ersten dominanten Frequenz oder der zweiten dominanten Frequenz oder beidem, wobei:
die Datenverarbeitungseinheit (4) ferner dafür konfiguriert ist, einen Grad der Ähnlichkeit zwischen der ersten dominanten Frequenz und der zweiten dominanten Frequenz zu bestimmen; und
die Datenverarbeitungseinheit (4) ferner dafür konfiguriert ist, die Ausgabe, die die Frequenz des periodischen physiologischen Prozesses darstellt, in Abhängigkeit von dem bestimmten Ähnlichkeitsgrad selektiv bereitzustellen.

## Revendications

1. Procédé informatisé de surveillance d'un sujet humain ou animal, comprenant :
(a) la réception de données de mesure représentant une série temporelle de mesures sur un sujet effectuées par un système de capteur (5) ;
(b) le traitement des données de mesure pour représenter les données de mesure comme une expansion mathématique comprenant une pluralité de composants d'expansion et une pluralité correspondante de coefficients d'expansion représentant la force respective de chaque composante d'expansion ;
(c1) la formation d'une première reconstruction partielle des données de mesure en utilisant un premier sous-ensemble des composants d'expansion et des coefficients d'expansion correspondants ;
(c2) la formation d'une deuxième reconstruction partielle des données de mesure en utilisant un deuxième sous-ensemble des composants d'expansion et des coefficients d'expansion correspondants, le deuxième sous-ensemble étant différent du premier sous-ensemble ;
(d1) l'exécution d'une première analyse spectrale sur la première reconstruction partielle pour déterminer une première fréquence dominante, la première fréquence dominante représentant une composante de fréquence de plus grande amplitude dans la première reconstruction partielle ;
(d2) l'exécution d'une deuxième analyse spectrale sur la deuxième reconstruction partielle pour déterminer une deuxième fréquence dominante, la deuxième fréquence dominante représentant une composante de fréquence de plus grande amplitude dans la deuxième reconstruction partielle ; et
(e) la fourniture d'une sortie représentant une fréquence d'un processus physiologique périodique basé sur l'une ou les deux de la première fréquence dominante et de la deuxième fréquence dominante, dans lequel :
le procédé comprend en outre la détermination d'un degré de similarité entre la première fréquence dominante et la deuxième fréquence dominante ; et
la sortie représentant la fréquence du processus physiologique périodique est fournie sélectivement en fonction du degré de similarité déterminé.

2. Procédé selon la revendication 1, dans lequel le système de capteur (5) comprend un ou plusieurs accéléromètres.

3. Procédé selon la revendication 1 ou 2, dans lequel la fréquence du processus physiologique périodique comprend un rythme respiratoire.

4. Procédé selon l'une quelconque des revendications précédentes, dans lequel les étapes (a) à (d2) sont répétées pour plusieurs unités des données de mesure, et l'étape (e) comprend la sortie de la fréquence du processus physiologique périodique uniquement pour les unités des données de mesure pour lesquelles le degré déterminé de similarité entre la première fréquence dominante et la deuxième fréquence dominante est supérieur à un seuil prédéterminé.

5. Procédé selon l'une quelconque des revendications précédentes, dans lequel chaque composant d'expansion comprend un vecteur propre et chaque coefficient d'expansion comprend une valeur propre.

6. Procédé selon la revendication 5, dans lequel les vecteurs propres et les valeurs propres sont déterminés en utilisant une analyse de spectre singulier.

7. Procédé selon l'une quelconque des revendications précédentes, dans lequel le premier sous-ensemble de composants d'expansion comprend les *N* composants ayant respectivement les *N* plus grands coefficients d'expansion, dans lequel de préférence 2 ≤ *N* ≤ 5.

8. Procédé selon la revendication 7, dans lequel le deuxième sous-ensemble de composants d'expansion ne comprend pas le composant d'expansion ayant le plus grand coefficient d'expansion.

9. Procédé selon la revendication 8, dans lequel le deuxième sous-ensemble de composants d'expansion comprend les composants d'expansion correspondant aux N plus grands coefficients d'expansion à l'exception du plus grand coefficient d'expansion.

10. Procédé selon l'une quelconque des revendications précédentes, dans lequel les données de mesure sont traitées pour éliminer au moins partiellement le bruit des données de mesure dans une étape de filtrage avant le traitement pour représenter les données de mesure sous la forme d'une expansion mathématique, de préférence dans lequel l'étape de filtrage comprend l'application un filtre passe-bas ou un enrichisseur de ligne adaptatif.

11. Procédé selon la revendication 10, dans lequel :
les données de mesure comprennent plusieurs canaux, chaque canal représentant des mesures effectuées en utilisant un mode de mesure différent ; et
le procédé comprend la détermination d'un rapport signal sur bruit des données de mesure dans chaque canal, après l'étape de filtrage, et l'utilisation du rapport signal sur bruit déterminé pour sélectionner l'un des canaux à utiliser dans les étapes suivantes pour fournir la sortie représentant la fréquence du processus physiologique périodique.

12. Procédé selon la revendication 11, dans lequel les différents modes de mesure comprennent chacun des mesures d'accélération par rapport à un axe respectif différent.

13. Programme informatique comprenant des instructions lisibles par ordinateur qui amènent un ordinateur à exécuter le procédé selon l'une quelconque des revendications précédentes.

14. Support lisible par ordinateur stockant le programme informatique selon la revendication 13.

15. Appareil (1) de surveillance d'un sujet humain ou animal, comprenant :
une unité de réception de données (3) configurée pour recevoir des données de mesure représentant une série temporelle de mesures sur un sujet effectuées par un système de capteur (5) ;
une unité de traitement de données (4) configurée pour :
traiter les données de mesure pour représenter les données de mesure comme une expansion mathématique comprenant une pluralité de composants d'expansion et une pluralité correspondante de coefficients d'expansion représentant la force respective de chaque composant d'expansion ;
former une première reconstruction partielle des données de mesure en utilisant un premier sous-ensemble des composants d'expansion et des coefficients d'expansion correspondants ;
former une deuxième reconstruction partielle des données de mesure en utilisant un deuxième sous-ensemble des composants d'expansion et des coefficients d'expansion correspondants, le deuxième sous-ensemble étant différent du premier sous-ensemble ;
effectuer une première analyse spectrale sur la première reconstruction partielle pour déterminer une première fréquence dominante, la première fréquence dominante représentant une composante de fréquence de plus grande amplitude dans la première reconstruction partielle ;
effectuer une deuxième analyse spectrale sur la deuxième reconstruction partielle pour déterminer une deuxième fréquence dominante, la deuxième fréquence dominante représentant une composante de fréquence de plus grande amplitude dans la deuxième reconstruction partielle ; et
fournir une sortie représentant une fréquence d'un processus physiologique périodique basé sur l'une ou les deux de la première fréquence dominante et de la deuxième fréquence dominante, dans lequel :
l'unité de traitement de données (4) est en outre configurée pour déterminer un degré de similarité entre la première fréquence dominante fréquence et la deuxième fréquence dominante ; et
l'unité de traitement de données (4) est en outre configurée pour fournir sélectivement la sortie représentant la fréquence du processus physiologique périodique en fonction du degré de similarité déterminé.
